# EUROPÄISCHE PATENTSCHRIFT

(11) **EP 3 225 271 B1**
(45) Veröffentlichungstag und Bekanntmachung des Hinweises auf die Patenterteilung: **04.09.2019**
(21) Anmeldenummer: 16163201.3
(22) Anmeldetag: 31.03.2016
(51) Int. Cl.: A61M 11/02, A61M 11/06, A61M 16/00, F04B 45/06, F04B 49/03, F16K 17/164, A61M 15/00, A61M 16/20, F16K 15/14

(54) **INHALIERVORRICHTUNG MIT ÜBERDRUCKVENTIL**
INHALATION DEVICE WITH PRESSURE-RELIEF VALVE
APPAREIL D'INHALATION AVEC UNE SOUPAPE DE SURPRESSION

(43) Veröffentlichungstag der Anmeldung: 04.10.2017
(73) Patentinhaber: PARI GmbH Spezialisten für effektive Inhalation, 82319 Starnberg (DE)
(72) Erfinder: Seidl, Christian, 81829 München (DE)
(74) Vertreter: Hoffmann Eitle

(56) Entgegenhaltungen:
- EP-A1- 0 049 316
- GB-A- 2 050 575
- US-A- 2 657 899
- US-A1- 2009 286 030
- US-A1- 2016 067 431

## Beschreibung

Die Erfindung betrifft eine Inhaliervorrichtung mit einem Kompressor und einem Aerosolerzeuger sowie einem Überdruckventil gemäß den beigefügten Ansprüchen.

Inhaliervorrichtungen mit einem Kompressor und einem Aerosolerzeuger sind im Stand der Technik bekannt und beispielsweise in EP 0170 715 B1 beschrieben.

EP 0 170 715 B1 beschreibt eine Vorrichtung zum Zerstäuben, Verteilen und Vermischen von flüssigen und pulverförmigen Stoffen mittels eines Druckgasstromes, insbesondere für die Erzeugung von Aerosolen für Inhalationszwecke. Als Druckgasquelle ist ein Kompressor angegeben, der einen erforderlichen Überdruck erzeugt (ca. 0,6 bar).

US 9,046,092 B2 beschreibt einen Kompressor mit zwei voneinander getrennten Kompressionsräumen.

US 2016/0067431 A1 offenbart eine Vorrichtung mit einer Maske oder einem Mundstück, einer Zuführung zum Zuführen von Luft zu der Maske oder dem Mundstück sowie einem Druckmesser, der in Fluidverbindung mit der Maske oder dem Mundstück angeordnet ist, sowie eine Einrichtung zum Erzeugen von pneumatischen Vibrationen in der Luft, die der Maske oder dem Mundstück zugeführt wird.

US 2009/286030 offenbart ein Überdruckventil mit einem elastischen Schlauchelement.

Es ergibt sich die Aufgabe, eine verbesserte Inhaliervorrichtung mit einem Kompressor und einem Aerosolerzeuger bereitzustellen.

Die Aufgabe wird gelöst durch eine Inhaliervorrichtung gemäß Anspruch 1, und zwar eine Inhaliervorrichtung mit einem Kompressor, einem Aerosolerzeuger und einem Überdruckventil, wobei der Kompressor eingerichtet ist, ein komprimiertes Gas für den Aerosolerzeuger bereitzustellen und das Überdruckventil eingerichtet ist, einen Druck in der Inhaliervorrichtung zu begrenzen. Des Weiteren ist erfindungsgemäß der Verwendungsanspruch 10 vorgesehen.

Durch das Vorsehen des Überdruckventils kann ein für den Aerosolerzeuger optimaler Druck bereitgestellt werden - es ist nicht nötig, aus Sicherheitsgründen einen niedrigeren Druck zu wählen. Durch das Vorsehen des Überdruckventils kann vermieden werden, dass hohe Drücke entstehen, die Gefährdungen eines Anwenders oder Beschädigungen der Inhaliervorrichtung verursachen könnten. So kann ein Aerosol mit optimierten Eigenschaften, vorzugsweise einem Druck von 1,0 bis 2,0 bar bereitgestellt werden. Ein Überdruck kann dazu führen, dass ein Schlauchende sich von einem Schlauchanschluss löst. Da das Überdruckventil das Entstehen von Überdrücken verhindern kann, kann die Verwendung eines Überdruckventils es ermöglichen, auf Befestigungsmittel, wie Kabelbinder oder Schlauchschellen an einem Schlauchende, das mit einem Schlauchanschluss verbunden ist, zu verzichten.

Eine Inhaliervorrichtung ist eine Vorrichtung, die zur Durchführung einer Inhalationstherapie geeignet ist.

Der Kompressor oder Verdichter ist eine Fluidenergiemaschine, die zum Komprimieren von Gasen geeignet ist. Der Kompressor umfasst vorzugsweise einen Rotationsverdichter, Schraubenverdichter, Kolbenverdichter, Taumelscheibenverdichter, Taumelkolbenverdichter oder Membranverdichter.

Ein Aerosolerzeuger weist bevorzugt einen Vernebler, einen Zerstäuber, einen Befeuchter, einen Druckluftvernebler, einen Luftzerstäuber, einen elektronischen Vernebler, einen elektrohydrodynamischen Vernebler, einen elektrostatischen Vernebler, einen Düsenvernebler, eine Zweistoffdüse, einen Atomizer oder eine Kombination davon auf. In einer Ausführungsform ist der Aerosolerzeuger für den Einsatz mit Beatmungsvorrichtungen eingerichtet.

Aerosole sind Gemische aus festen oder flüssigen Schwebeteilchen und einem Gas. Aerosole sind bevorzugt zur Applikation auf oder in Teile des menschlichen oder tierischen Körpers, wie Haut, Körperhöhlen, Körperöffnungen, Nase, Nasennebenhöhlen, Kieferhöhle, Stirnhöhle, Keilbeinhöhle, Siebbeinzellen, Rachen, Kehlkopf, Luftröhre, Lunge, Stammbronchus, Bronchien, Bronchiolen, Lungenbläschen, Gelenke oder Bauchraum vorgesehen. Aerosole können eingesetzt werden, um Krankheiten von Menschen und Tieren zu diagnostizieren, vorzubeugen, zu therapieren oder um Menschen oder Tiere gegen Krankheiten zu immunisieren.

Überdruckventile oder Sicherheitsventile sind dazu eingerichtet, einen unzulässigen Druckanstieg, der zu einer Gefährdung eines Anwenders oder einer Schädigung eines Bauteils führen kann, zu verhindern. Überdruckventile sind dazu eingerichtet, bei Überschreiten eines Ansprechdruckes Fluide, Gase, Dämpfe oder Flüssigkeiten aus dem druckbelasteten Bereich abzuleiten. Das Überdruckventil weist in einer Ausführungsform ein Proportionalventil, Vollhubsicherheitsventil, Normalsicherheitsventil, federbelastetes Sicherheitsventil, gewichtsbelastetes Sicherheitsventil, mediumbelastetes Sicherheitsventil, direkt wirkendes Sicherheitsventil, gesteuertes Sicherheitsventil oder eine Kombination davon auf.

Das komprimierte Gas weist vorzugsweise komprimierte Luft auf.

Es ist zweckmäßig, wenn das Überdruckventil stromabwärts des Kompressors vorgesehen ist.

In einer Ausführungsform ist das Überdruckventil dazu eingerichtet, einen Druck innerhalb des Kompressors, des Aerosolerzeugers oder eines Schlauchs, zu begrenzen. Der Schlauch ist vorzugsweise dazu eingerichtet, komprimiertes Gas von dem Kompressor zu dem Aerosolerzeuger leiten zu können. Zweckmäßigerweise ist das Überdruckventil eingerichtet, einen mechanischen Schutz aller unter Druck stehender Bauteile zu bieten. Das Überdruckventil kann einen Schutz gegen einen schädlichen Druck bieten.

Vorzugsweise ist das Überdruckventil benachbart zu einem Auslass des Kompressors angeordnet. Der Kompressor kann so besonders gut vor einer Überlastung durch einen zu hohen Druck geschützt werden.

In einer Ausführungsform ist das Überdruckventil an dem Schlauch oder an dem Kompressor vorgesehen. Bevorzugt ist das Überdruckventil an einem Auslass des Kompressors, benachbart zu einem Ende des Schlauchs oder an einem Schlauchanschluss vorgesehen.

Ein Schlauchanschluss umfasst in einer Ausführungsform eine Schlauchtülle, eine Lufttülle, einen Luftstutzen oder einen Rohrstutzen und ist dazu eingerichtet, mit einem Auslass eines Kompressors fest oder lösbar verbunden zu werden. Es ist bevorzugt, wenn der Schlauchanschluss einen kreisförmigen Querschnitt aufweist. So kann der Schlauchanschluss auf einfache Weise mit gängigen Schläuchen kombiniert werden. Vorzugsweise weist der Schlauchanschluss einen Kunststoff auf. Es ist besonders bevorzugt, wenn der Schlauchanschluss vollständig aus einem Kunststoff besteht.

Vorzugsweise weist der Kompressor einen Kolben auf. Ein Kompressor mit einem Kolben kann durch die Kombination mit einem Überdruckventil derart ausgelegt werden, dass der Kompressor einen besonders hohen Wirkungsgrad hat.

Ein Kompressor mit einem Kolben oder Kolbenverdichter arbeitet zweckmäßigerweise nach dem Verdrängerprinzip. Vorzugsweise ist der Kompressor mit einem Kolben eingerichtet, ein Gas in einem Volumen zu kapseln, zu verdichten und wieder auszustoßen. Vorzugsweise ist der Kompressor mit einem Kolben eingerichtet, ein Gas in einem Inneren eines Zylinders durch eine Kolbenbewegung zu verdichten.

Vorzugsweise ist der Kolben ein Taumelkolben. So kann der Kompressor auf besonders einfache Weise bereitgestellt werden.

In einer Ausführungsform ist der Taumelkolben starr mit einer Schubstange verbunden, welche ein Ende aufweist, das derart bewegt werden kann, dass der Taumelkolben eine Hub- und eine Drehbewegung oder eine Hub- und eine Kippbewegung ausführt. Die Bewegung des Endes der Schubstange ist vorzugsweise eine Kreisbewegung. Die Kreisbewegung wird zweckmäßigerweise durch eine exzentrische Anbindung des Endes der Schubstange an eine Welle ermöglicht. Dabei ist der Taumelkolben zweckmäßigerweise in einem Zylinder angeordnet.

In einer bevorzugten Ausführungsform weist ein Schlauchanschluss in einem vorderen Bereich eine Dichtfläche auf, auf der der Schlauch oder Druckschlauch bei Anliegen eines Arbeitsdrucks abdichtet und vorzugsweise vollkommen anliegt. In einer bevorzugten Ausführungsform ist eine Vertiefung, wie eine Kerbe oder eine Nut in einen Mittelbereich oder Mittelteil des Schlauchanschlusses derart eingebracht, dass ein über dem Arbeitsdruck liegender Überdruck hindurch entweichen kann. Der Schlauch ist vorzugsweise dazu eingerichtet, sich im Überdruckfall, bei Vorliegen des Überdrucks, der über dem Arbeitsdruck liegt, aufzublähen, so dass er von der Dichtfläche abgehoben wird und das Gas aus dem Schlauch zu der Nut gelangen kann. Im Mittelbereich des Schlauchanschlusses ist vorzugsweise eine Verzahnung vorgesehen, die dazu eingerichtet ist, den Schlauch auch im Überdruckfall von einem Abgleiten abzuhalten oder am Schlauchanschluss festzuhalten.

In einer Ausführungsform weist der Schlauch einen kreisförmigen oder runden Querschnitt auf. Zweckmäßigerweise weist der Schlauch einen Kunststoff auf. In einer Ausführungsform ist der Schlauch ausschließlich aus Kunststoff gefertigt.

Vorzugsweise weist der Schlauchanschluss einen ovalen oder elliptischen Querschnitt, besonders bevorzugt einen kreisförmigen oder runden Querschnitt auf.

In einer Ausführungsform ist der Schlauchanschluss mit dem Kompressor fest verbunden. Es ist bevorzugt, wenn der Schlauchanschluss mit dem Schlauch fest verbunden ist.

Vorzugsweise ist im Bereich der Vertiefung eine Schlauchbefestigung um den Schlauch herum vorgesehen. Dadurch kann die Vertiefung unterhalb des Grenzdrucks optimal abgedichtet werden.

In einer Ausführungsform umfasst die Schlauchbefestigung eine Klemme, einen Kabelbinder oder eine Schlauchschelle. In einer Ausführungsform ist ein Schlauch einer internen Verschlauchung so kurz und die Schlauchanschlüsse liegen einander mit einem derart geringen Versatz gegenüber, dass der Schlauch bei einem Überdruck an den Schlauchanschlüssen befestigt bleibt. Eine Schlauchbefestigung, wie eine Klemme, ein Kabelbinder oder eine Schlauchschelle ist nicht vorgesehen.

Vorzugsweise ist an dem Schlauchanschluss eine Aufweitung vorhanden. So kann der Schlauch besonders sicher mit dem Schlauchanschluss verbunden werden. Ein Abrutschen des Schlauchs von dem Schlauchanschluss kann verhindert werden.

Zweckmäßigerweise ist die Aufweitung derart ausgebildet, dass sie dazu geeignet ist, von Innen gegen den Schlauch zu drücken.

In einer Ausführungsform verläuft die Vertiefung durch die Aufweitung hindurch.

Es ist besonders zweckmäßig, wenn die Aufweitung eine Kante aufweist. Die Kante ist vorzugsweise an dem größten Querschnitt der Aufweitung vorgesehen. In einer Ausführungsform ist die Aufweitung in Form einer Verzahnung ausgebildet.

Vorzugsweise beinhaltet der Kompressor einen Zylinder, der einen Kunststoff aufweist. Dadurch kann der Kompressor auf besonders einfache Weise gefertigt werden. Vorzugsweise weist der Kompressor einen Kolben oder ein Gehäuse mit einem Kunststoffanteil oder bestehend aus Kunststoff auf. Es ist besonders bevorzugt, wenn der Kompressor größtenteils aus einem oder mehreren Kunststoffen gefertigt ist. In einer Ausführungsform weist der Kompressor überwiegend nichtmetallische Werkstoffe auf. In einer Ausführungsform weist der Zylinder keinen metallischen Werkstoff auf. In einer Ausführungsform weist der Kolben keinen metallischen Werkstoff auf.

Vorzugsweise ist der Kompressor dazu vorgesehen, einen Arbeitsdruck zwischen 1 und 3 bar zu erzeugen. Dadurch ist das komprimierte Gas besonders dazu geeignet dem Aerosolerzeuger zugeführt zu werden. Besonders bevorzugt liegt der Druck des komprimierten Gases bei Benutzung der Inhaliervorrichtung zwischen 1,0 und 2,0 bar. Es ist besonders zweckmäßig, wenn der Kompressor dazu eingerichtet ist, einen Verdichtungsgrad zwischen 1:2 und 1:20, bevorzugt zwischen 1:5 und 1:10, idealerweise von 1:8 zu erzeugen. Durch Wahl des Verdichtungsverhältnisses kann der volumetrische Wirkungsgrad des Kompressors beeinflusst werden.

Ein besserer Wirkungsgrad ermöglicht die Verwendung kleinerer oder günstigerer Motoren für den Kompressor. Ein höher verdichtender Kompressor erzeugt zweckmäßigerweise höhere Staudrücke. Bei einer Blockade einer Düse des Aerosolerzeugers, einem Zuhalten eines Luftausganges oder Knicken des Schlauches können sehr hohe Drücke, Kräfte und Temperaturen an Bauteilen des Kompressors, einer Druckluftleitung und an dem Aerosolerzeuger entstehen, die Gefährdungen und Beschädigungen verursachen können. Der Schlauch kann platzen und es kann ein Bauteil ausfallen, insbesondere kann ein Kolben, der als Topfmanschette ausgebildet ist, ausfallen. Durch die Verwendung eines Überdruckventils können diese Probleme verhindert werden, so dass der Kompressorwirkungsgrad gesteigert werden kann. Es kann ein Kompressor mit hohem Verdichtungsverhältnis und hohen Drücken bereitgestellt werden, ohne dass beispielsweise bei einer Blockade oder Verstopfung einer Düse des Aerosolerzeugers oder bei einem Knicken eines Schlauchs schädliche Überdrücke entstehen können oder der Kompressor nicht anläuft, der Stromverbrauch des Kompressors ansteigt und der Kompressor sich stark erwärmt. Defekte an Stromsicherungen können vermieden werden.

In einer Ausführungsform ist ein vergleichsweise großer Ansaugquerschnitt gewählt.

Die Aufgabe wird auch gelöst durch ein Überdruckventil für eine Inhaliervorrichtung mit einem Kompressor und einem Aerosolerzeuger, wobei der Kompressor eingerichtet ist, ein komprimiertes Gas für den Aerosolerzeuger bereitzustellen, und das Überdruckventil einen Schlauchabschnitt und einen Schlauchanschluss aufweist, wobei der Schlauchanschluss eine Vertiefung umfasst, der Schlauch unterhalb eines Grenzdrucks in einem Inneren des Schlauchs die Vertiefung abdichtet und sich oberhalb des Grenzdrucks in dem Inneren des Schlauchs derart aufweitet, dass die Vertiefung zu einer Umgebung geöffnet ist, so dass Gas aus dem Schlauch durch die Vertiefung in die Umgebung entweichen kann.

Im Folgenden wird die Erfindung anhand von Ausführungsbeispielen unter Bezugnahme auf die beigefügten Figuren genauer beschrieben. Es zeigen:
Figur 1 eine Schemazeichnung einer Inhaliervorrichtung mit einem Kompressor, einem Aerosolerzeuger und einem Überdruckventil;
Figur 2 ein Überdruckventil;
Figur 3 das in Figur 2 gezeigte Überdruckventil bei Vorliegen eines hohen Drucks;
Figur 4 ein Überdruckventil mit einer Haltekante;
Figur 5 das in Figur 4 gezeigte Überdruckventil bei Vorliegen eines hohen Drucks;
Figur 6 ein Überdruckventil mit einer Haltekante und einer Schelle; und
Figur 7 das in Figur 6 gezeigte Überdruckventil bei Vorliegen eines hohen Drucks.

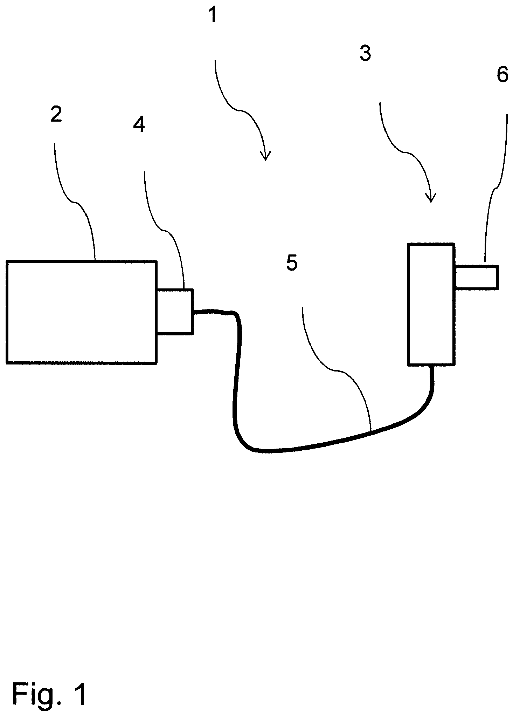

Figur 1 zeigt eine Schemazeichnung einer Inhaliervorrichtung 1 mit einem Taumelkolbenkompressor 2, einem Aerosolerzeuger 3 und einem Überdruckventil 4. Das Überdruckventil 4 ist an dem Taumelkolbenkompressor 2 angeordnet. Der Aerosolerzeuger 3 ist ein Düsenvernebler 3 und ist über eine Druckluftleitung 5 mit dem Überdruckventil 4 und dem Taumelkolbenkompressor 2 verbunden. An dem Düsenvernebler 3 ist ein Mundstück 6 vorgesehen.

Bei Benutzung der Inhaliervorrichtung 1 erzeugt der Taumelkolbenkompressor 2 Druckluft, die über die Druckluftleitung 5 zu dem Düsenvernebler 3 geleitet wird. Der Düsenvernebler 3 erzeugt mit Hilfe der Druckluft und einer Flüssigkeit ein Aerosol. Das Aerosol kann von einem Benutzer durch das Mundstück 6 inhaliert werden.

Das Überdruckventil 4 begrenzt den relativen Druck zwischen der Inhaliervorrichtung 1 und der Umgebung auf 10 bar.

Wenn der relative Druck diesen Wert übersteigt, stellt das Überdruckventil 4 eine Verbindung zwischen einem Inneren der Druckluftleitung 5 und der Umgebung her. Dadurch kann Luft aus der Inhaliervorrichtung 1 in die Umgebung entweichen und der relative Druck in der Inhaliervorrichtung 1 sinkt ab.

Gefährdungen des Benutzers oder sich in der Nähe befindlicher Personen und Beschädigungen der Inhaliervorrichtung 1 können so vermieden werden. Der Kompressor 2 kann dazu ausgelegt werden, einen hohen Druck zu liefern. Es ist nicht nötig, den Kompressor 2 dazu auszulegen, einen niedrigen Druck zu liefern, um Gefährdungen oder Beschädigungen zu vermeiden. Die Inhaliervorrichtung 1 kann so ausgelegt werden, dass sie einen hohen Wirkungsgrad hat.

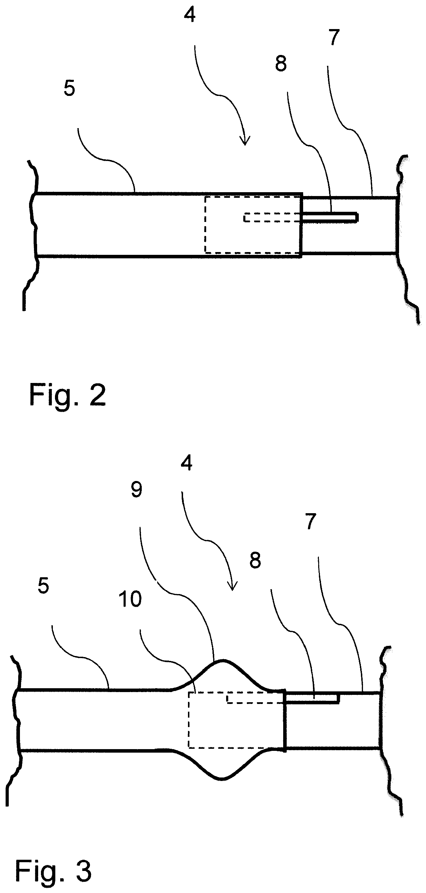

Figur 2 zeigt ein Überdruckventil 4, wie es in einer Inhaliervorrichtung 1, beispielweise in der in Figur 1 gezeigten Inhaliervorrichtung 1, eingesetzt werden kann. Das Überdruckventil 4 ist in einem Zustand gezeigt, den es bei Vorliegen eines Betriebsdrucks oder in drucklosem Zustand einnimmt.

Das Überdruckventil 4 weist eine Schlauchtülle 7, eine Nut 8 und ein Ende eines Schlauchs 5 auf. Die Schlauchtülle 7 kann mit einem Kompressor 2 verbunden werden. Der Schlauch 5 kann mit einem Vernebler 3 verbunden werden. Bei Vorliegen eines Betriebsdrucks oder bei ausgeschaltetem Kompressor 2 liegt der Schlauch 5 glatt an der Schlauchtülle 7 an. Das Innere des Schlauchs 5 ist derart gegen die Umgebung abgedichtet, dass Druckluft aus dem Inneren des Schlauchs 5 nicht in die Umgebung entweichen kann. Die Nut 8 ist mit der Umgebung, jedoch nicht mit dem Inneren des Schlauchs 5 oder der Schlauchtülle 7 verbunden.

Figur 3 zeigt das in Figur 2 gezeigte Überdruckventil 4 bei Vorliegen eines hohen Innendrucks oder Drucks in dem Inneren des Schlauchs 5, der einen vorgesehenen Betriebsdruck übersteigt. Der relative Druck im Inneren des Schlauchs 5 zur Umgebung beträgt 3 bar.

Durch den hohen relativen Druck ist der Schlauch 5 derart aufgebläht, dass er stellenweise von der Schlauchtülle 7 abgehoben ist. Dadurch ist eine Verbindung zwischen dem Inneren des Schlauchs 5 und der Nut 8 vorhanden. Druckluft kann aus dem Inneren des Schlauchs 5 durch den aufgeblähten Bereich 9 an einer Außenseite 10 der Schlauchtülle 7 vorbei durch die Nut 8 in die Umgebung entweichen. So kann ein Druck im Inneren des Schlauchs 5 abgesenkt werden oder ein weiteres Ansteigen des Drucks verhindert werden. Am auslassseitigen Bereich der Nut 8 ist der Innendruck nicht in der Lage, den Schlauch 5 von der Schlauchtülle 7 abzuheben, da der Druck nach Herstellung eines Durchlasses aus dem Inneren des Schlauchs 5 in die Nut 8 ausreichend abgebaut wird. Der Schlauch 5 rutscht daher auch bei einem hohen relativen Druck nicht von der Schlauchtülle 7 ab.

Wenn der Innendruck abnimmt und ein vorgesehener relativer Druck erreicht ist, nimmt das Überdruckventil 4 wieder den in Figur 2 gezeigten Zustand ein. Es kann dann keine Druckluft mehr durch die Nut 8 in die Umgebung entweichen.

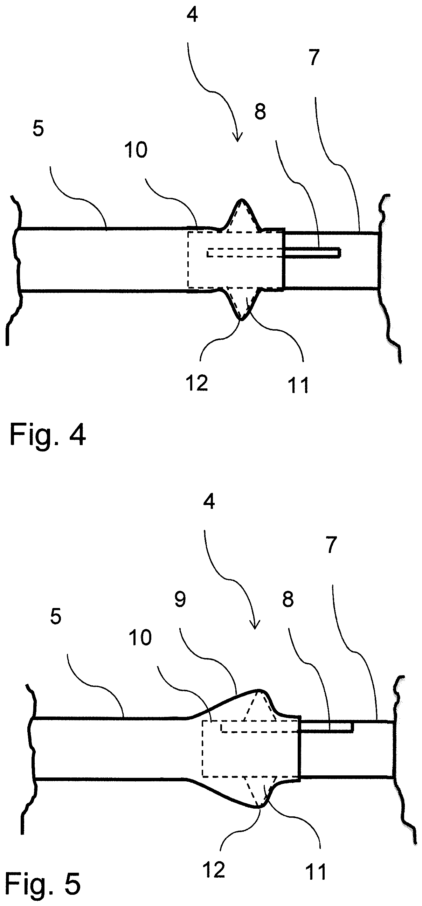

Figur 4 zeigt ein Überdruckventil 4 mit einer Haltekante 11. Das Überdruckventil 4 ist entsprechend dem in den Figuren 2 und 3 gezeigten Überdruckventil 4 aufgebaut. Um den Schlauch 5 besser gegen ein Abrutschen von der Schlauchtülle 7 zu sichern, ist die Haltekante 11 vorgesehen. Die Haltekante 11 ist ein Bereich der Schlauchtülle 7, der einen größeren Durchmesser aufweist als die in Längsrichtung benachbarten Bereiche der Schlauchtülle 7. An der Stelle mit dem größten Durchmesser weist die Haltekante 11 eine scharfe Kante 12 auf.

Bei Vorliegen eines Betriebsdrucks oder im drucklosen Zustand liegt der Schlauch 5 dicht an der Haltekante 11 an. Dadurch ist die Verbindung zwischen Schlauch 5 und Haltekante 11 besonders fest.

Figur 5 zeigt das in Figur 4 gezeigte Überdruckventil 4 bei Vorliegen eines hohen Drucks im Inneren des Schlauchs 5. Wie bei dem in Figur 3 beschriebenen Überdruckventil 4 entsteht ein aufgeblähter Bereich 9, so dass Druckluft aus dem Inneren des Schlauchs 5 durch den aufgeblähten Bereich 9 an einer Außenseite 10 der Schlauchtülle 7 vorbei durch die Nut 8 in die Umgebung entweichen kann. Die Haltekante 11 sichert den Schlauch 5 gegen Abrutschen. Die Haltekante 11 definiert den Bereich, in dem sich der Schlauch 5 aufblähen kann. An der Druckseite, oder wirksam dem Kompressor 2 zugewandten Seite der Haltekante kann sich der Schlauch 5 aufblähen. Der Bereich des Schlauchs 5, der sich auf der Seite der Haltekante 11 befindet, die dem benachbarten Ende des Schlauchs 5 zugewandt ist, liegt glatt an Haltekante 11 und Schlauchtülle 7 an.

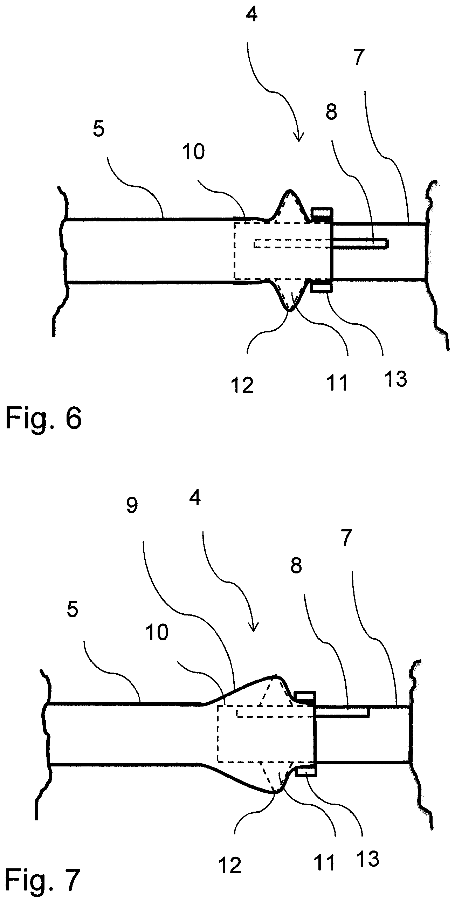

Figur 6 zeigt ein Überdruckventil 4 mit einer Haltekante 11 und einer Schlauchklemme 13 in drucklosem Zustand. Das in Figur 6 gezeigte Überdruckventil 4 entspricht dem in den Figuren 4 und 5 gezeigten Überdruckventil 4 mit der Ausnahme, dass eine Schlauchklemme 13 vorgesehen ist. Die Schlauchklemme 13 stellt einen zusätzlichen Schutz gegen ein Abrutschen des Schlauchs 5 dar. Sie ist benachbart zu der Haltekante 11 auf der dem benachbarten Ende des Schlauchs 5 zugewandten Seite der Haltekante 11 vorgesehen. Die Schlauchklemme 13 erstreckt sich um Schlauch 5 und Schlauchtülle 7 herum. Die Schlauchklemme 13 ist in einem Bereich der Schlauchtülle 7 vorgesehen, durch den die Nut 8 verläuft.

Figur 7 zeigt das in Figur 6 gezeigte Überdruckventil 4 bei Vorliegen eines hohen Drucks. Die Schlauchklemme 13 kann ein Abrutschen des Schlauchs verhindern. Außerdem kann sie verhindern, dass der Durchlass durch ein Aufblähen des Schlauchs 5 größer wird als durch den Querschnitt der Nut 8 vorgesehen.

### BEZUGSZEICHENLISTE

- 1: Inhaliervorrichtung
- 2: Kompressor
- 3: Aerosolerzeuger
- 4: Überdruckventil
- 5: Schlauch
- 6: Mundstück
- 7: Schlauchtülle
- 8: Nut
- 9: aufgeblähter Bereich
- 10: Außenseite der Schlauchtülle
- 11: Haltekante
- 12: scharfe Kante
- 13: Schlauchklemme

## Patentansprüche

1. Inhaliervorrichtung (1) mit einem Kompressor (2), einem Aerosolerzeuger (3) und einem Überdruckventil (4), wobei der Kompressor (2) eingerichtet ist, ein komprimiertes Gas für den Aerosolerzeuger (3) bereitzustellen und das Überdruckventil (4) eingerichtet ist, einen Druck in der Inhaliervorrichtung (1) zu begrenzen, wobei
das Überdruckventil (4) einen Schlauchabschnitt (5) und eine Vertiefung (8) in einem Schlauchanschluss (7) aufweist und derart eingerichtet ist, dass der Schlauch (5) unterhalb eines Grenzdrucks in einem Inneren des Schlauchs (5) die Vertiefung (8) abdichtet und sich oberhalb des Grenzdrucks in dem Inneren des Schlauchs (5) derart aufweitet, dass die Vertiefung (8) zu einer Umgebung geöffnet ist, so dass Gas aus dem Schlauch (5) durch die Vertiefung (8) in die Umgebung entweichen kann.

2. Inhaliervorrichtung (1) nach Anspruch 1, **dadurch gekennzeichnet, dass** das Überdruckventil (4) benachbart zu einem Auslass des Kompressors (2) angeordnet ist.

3. Inhaliervorrichtung (1) nach einem der vorhergehenden Ansprüche, **dadurch gekennzeichnet, dass** der Kompressor (2) einen Kolben aufweist.

4. Inhaliervorrichtung (1) nach Anspruch 3, **dadurch gekennzeichnet, dass** der Kolben ein Taumelkolben ist.

5. Inhaliervorrichtung nach einem der vorhergehenden Ansprüche, **dadurch gekennzeichnet, dass** im Bereich der Vertiefung (8) eine Schlauchbefestigung (13) um den Schlauch (5) herum vorgesehen ist.

6. Inhaliervorrichtung (1) nach einem der vorhergehenden Ansprüche, **dadurch gekennzeichnet, dass** an dem Schlauchanschluss (7) eine Aufweitung (11, 12) vorhanden ist.

7. Inhaliervorrichtung (1) nach einem der vorhergehenden Ansprüche, **dadurch gekennzeichnet, dass** der Kompressor (2) einen Zylinder beinhaltet, der einen Kunststoff aufweist.

8. Inhaliervorrichtung (1) nach einem der vorhergehenden Ansprüche, **dadurch gekennzeichnet, dass** der Kompressor (2) dazu vorgesehen ist, einen Arbeitsdruck zwischen 1 und 3 bar zu erzeugen.

9. Verwendung eines Überdruckventils (4) in einer Inhaliervorrichtung (1) mit einem Kompressor (2) und einem Aerosolerzeuger (3), wobei der Kompressor (2) ein komprimiertes Gas für den Aerosolerzeuger (3) bereitstellt und das Überdruckventil (4) einen Druck in der Inhaliervorrichtung (1) begrenzt, wobei das Überdruckventil (4) einen Schlauchabschnitt (5) und eine Vertiefung (8) in einem Schlauchanschluss (7) aufweist, wobei der Schlauch (5) unterhalb eines Grenzdrucks in einem Inneren des Schlauchs (5) die Vertiefung (8) abdichtet und sich oberhalb des Grenzdrucks in dem Inneren des Schlauchs (5) derart aufweitet, dass die Vertiefung (8) zu einer Umgebung geöffnet ist, so dass Gas aus dem Schlauch (5) durch die Vertiefung (8) in die Umgebung entweicht.

## Claims

1. An inhalation device (1) comprising a compressor (2), an aerosol generator (3) and a pressure relief valve (4), wherein the compressor (2) is configured to provide a compressed gas for the aerosol generator (3) and the pressure relief valve (4) is configured to limit a pressure in the inhalation device (1), the pressure relief valve (4) comprising a hose section (5) and an indentation (8) in a hose connection nozzle (7), and being so configured that said hose (5) seals the indentation (8) below a limiting pressure in the interior of the hose (5) and expands above the limiting pressure in the interior of the hose (5) such that the indentation (8) is opened to the surroundings in order that gas can escape from the hose (5) into the surroundings via the indentation (8).

2. The inhalation device (1) according to claim 1, **characterised in that** the pressure relief valve (4) is disposed adjacent to an outlet of the compressor (2).

3. The inhalation device (1) according to one of the preceding claims, **characterised in that** the compressor (2) comprises a piston.

4. The inhalation device (1) according to claim 3, **characterised in that** the piston is a wobble piston.

5. The inhalation device according to one of the preceding claims, **characterised in that** in the region of the indentation (8), a hose fastening device (13) is provided around the hose (5).

6. The inhalation device (1) according to one of the preceding claims, **characterised in that** an expansion (11, 12) is provided on the hose connection nozzle (7).

7. The inhalation device (1) according to one of the preceding claims, **characterised in that** the compressor (2) includes a cylinder that comprises a plastic.

8. The inhalation device (1) according to one of the preceding claims, **characterised in that** the compressor (2) is provided to generate an operating pressure of between 1 and 3 bar.

9. Use of a pressure relief valve (4) in an inhalation device (1) comprising a compressor (2) and an aerosol generator (3), wherein the compressor (2) provides a compressed gas for the aerosol generator (3) and the pressure relief valve (4) limits a pressure in the inhalation device (1), the pressure relief valve (4) comprising a hose section (5) and an indentation (8) in a hose connection nozzle (7), said hose (5) sealing the indentation (8) below a limiting pressure in the interior of the hose (5) and expanding above the limiting pressure in the interior of the hose (5) such that the indentation (8) is opened to the surroundings in order that gas can escape from the hose (5) into the surroundings via the indentation (8).

## Revendications

1. Dispositif d'inhalation (1) avec un compresseur (2), un générateur d'aérosol (3) et une soupape de surpression (4), dans lequel le compresseur (2) est aménagé pour mettre à disposition un gaz comprimé pour le générateur d'aérosol (3) et la soupape de surpression (4) est aménagée pour limiter une pression dans le dispositif d'inhalation (1), dans lequel la soupape de surpression (4) présente une section de tuyau (5) et un évidement (8) dans un raccord de tuyau (7) et est aménagée de sorte que le tuyau (5) étanchéifie l'évidement (8) sous une pression limite dans un intérieur du tuyau (5) et s'élargit au-dessus de la pression limite à l'intérieur du tuyau (5) de sorte que l'évidement (8) est ouvert vers un environnement, de sorte que du gaz puisse s'échapper du tuyau (5) dans l'environnement à travers l'évidement (8).

2. Dispositif d'inhalation (1) selon la revendication 1, **caractérisé en ce que** la soupape de surpression (4) est agencée de manière adjacente à une sortie du compresseur (2).

3. Dispositif d'inhalation (1) selon l'une quelconque des revendications précédentes, **caractérisé en ce que** le compresseur (2) présente un piston.

4. Dispositif d'inhalation (1) selon la revendication 3, **caractérisé en ce que** le piston est un piston basculant.

5. Dispositif d'inhalation selon l'une quelconque des revendications précédentes, **caractérisé en ce qu'**une fixation de tuyau (13) est prévue autour du tuyau (5) dans la zone de l'évidement (8).

6. Dispositif d'inhalation (1) selon l'une quelconque des revendications précédentes, **caractérisé en ce qu'**un élargissement (11, 12) est présent au niveau du raccord de tuyau (7).

7. Dispositif d'inhalation (1) selon l'une quelconque des revendications précédentes, **caractérisé en ce que** le compresseur (2) contient un cylindre, qui présente un plastique.

8. Dispositif d'inhalation (1) selon l'une quelconque des revendications précédentes, **caractérisé en ce que** le compresseur (2) est prévu pour générer une pression de travail entre 1 et 3 bars.

9. Utilisation d'une soupape de surpression (4) dans un dispositif d'inhalation (1) avec un compresseur (2) et un générateur d'aérosol (3), dans lequel le compresseur (2) met à disposition un gaz comprimé pour le générateur d'aérosol (3) et la soupape de surpression (4) limite une pression dans le dispositif d'inhalation (1), dans lequel la soupape de surpression (4) présente une section de tuyau (5) et un évidement (8) dans un raccord de tuyau (7), dans laquelle le tuyau (5) étanchéifie l'évidement (8) sous une pression limite dans un intérieur du tuyau (5) et s'élargit au-dessus de la pression limite à l'intérieur du tuyau (5) de sorte que l'évidement (8) est ouvert vers un environnement, de sorte que du gaz s'échappe du tuyau (5) dans l'environnement à travers l'évidement (8).
